# EUROPÄISCHE PATENTSCHRIFT

(11) **EP 0 988 583 B1**
(45) Veröffentlichungstag und Bekanntmachung des Hinweises auf die Patenterteilung: **16.02.2005**
(21) Anmeldenummer: 99914427.2
(22) Anmeldetag: 24.02.1999
(51) Int. Cl.: G05B 19/406

(54) **SYSTEM UND VERFAHREN ZUR DIAGNOSE VON TRIEBWERKSZUSTÄNDEN**
SYSTEM AND METHOD FOR DIAGNOSING THE CONDITIONS OF A POWERPLANT
SYSTEME ET PROCEDE POUR LE DIAGNOSTIC D'ETATS DE GROUPES MOTOPROPULSEURS

(30) Priorität: 27.02.1998 DE 19808197
(43) Veröffentlichungstag der Anmeldung: 29.03.2000
(73) Patentinhaber: MTU Aero Engines GmbH, 80995 München (DE)
(72) Erfinder: MORENO-BARRAGAN, Jorge, D-80997 München (DE)
(74) Vertreter: Einsele, Rolf W.
(86) Internationale Anmeldenummer: PCT/DE1999/000491
(87) Internationale Veröffentlichungsnummer: WO 1999/044106

(56) Entgegenhaltungen:
- EP-A- 0 805 382
- DE-U- 29 700 028

## Beschreibung

Die Erfindung bezieht sich auf ein System und Verfahren zur Diagnose von Triebwerkszuständen.

Herkömmlich erfolgt die Diagnose von Triebwerkszuständen unter Verwendung von Schwingungssignalen aufgrund von Amplitudengrenzen, die aus der allgemeinen Erfahrung abgeleitet wurden, und/oder typischer Merkmale aus Schwingungssignaturen, der Erfahrung aus Ereignissen während der Entwicklungsphase bzw. der Erfahrung aus dem Zertifizierungsprozeß.

Es erfolgen in der Regel kostspielige und zeitintensive Modifikationen in der Serienproduktion.

Die Schwingungsdiagnose wird dabei von verschieden qualifizierten Spezialistenteams durchgeführt, ohne gezielten Erfahrungsaustausch zwischen Betreiber und Hersteller von Triebwerken und ohne systematische Erfassung und Auswertung von Fehlern, Begleiterscheinungen bzw. Anzeichen und deren Ursachen.

Somit besteht bei der bisher üblichen Schwingungsdiagnose von Triebwerkszuständen unter anderem das Problem, daß wenigen Meßpositionen nur eine begrenzte Menge von Informationen zur Auswertung gegenüber steht. Es gibt zwar aus der Entwicklungsphase Fehlerkataloge; diese sind aber meistens sehr lückenhaft. Der Einfluß einer großen Anzahl von Parametern, wie beispielsweise von Baustandards, Toleranzen, Größe und Position von Unwuchten, Temperaturauswirkungen, Leistungs- und Flugparametern usw., sowie Nichtlinearitäten und Meßungenauigkeiten bleibt weitgehend unberücksichtigt.

Daher können bei dieser Art von Schwingungsdiagnose gefährliche Schwingungsbedingungen während des Betriebs unerkannt weiterbestehen, es können größere Sekundärschäden durch zu späte Fehlererkennung auftreten und der Wartungsaufwand steigt, da zumeist eine Triebwerkszerlegung erforderlich ist.

Deshalb ist es Aufgabe der vorliegenden Erfindung, ein System und Verfahren zur Diagnose von Triebwerkszuständen zu schaffen, bei dem die Sicherheit durch ein Erkennen gefährlicher Schwingungsbedingungen erhöht wird, größere Sekundärschäden durch eine frühe Fehlererkennung vermieden werden, der Wartungsaufwand durch gezielte Elimination der Schwingungsursachen verringert wird und eine Wartung entsprechend dem aktuellen Triebwerkszustand (d.h. "On-Condition") erfolgt.

Diese Aufgabe wird durch die in den Patentansprüchen 1 bzw. 8 aufgezeigten Maßnahmen gelöst. In den Untersprüchen sind vorteilhafte Ausgestaltungen und Weiterbildungen angegeben.

In der nachfolgenden Beschreibung ist ein Ausführungsbeispiel der Erfindung in Verbindung mit der Zeichnung beschrieben.

Es zeigen:
Fig. 1A eine schematische Darstellung des Aufbaus eines Neuronalen Mehrschicht-Netzes zur Informationsverdichtung,
Fig. 1B eine schematische Darstellung des Aufbaus eines Neuronalen Mehrschicht-Netzes zur Informationsverknüpfung,
Fig. 1C einen Aufbau einer Neuroneneinheit, die in den Netzen gemäß Fig. 1A und Fig. 1B verwendet wird, und
Fig. 2 ein Blockschaltbild zur Veranschaulichung des Aufbaus des erfindungsgemäßen Systems zur Diagnose von Triebwerkszuständen.

Der Schwerpunkt der Erfindung liegt bei einem Verfahren und einer Vorrichtung für die Extraktion von Merkmalen aus unterschiedlichen Informationsquellen und deren Bearbeitung. Diese Merkmale, die den Triebwerkszustand umfassend charakterisieren, bilden den Kern des Systems zusammen mit einer Hintereinanderschaltung zweier Neuronaler Netze.

Zum Generieren von Schwingungsmustem (Trainingsdaten) zur Diagnose von Triebwerkszuständen kann sowohl eine Simulation als auch eine Messung verwendet werden. Beide Verfahren besitzen ihre Vor- und Nachteile, die im folgenden erläutert werden.

Bei einer Simulation ist es vorteilhaft, daß eine Analyse verschiedener vordefinierter Fehlerfälle und außerdem eine Kombination davon erfolgt. Die Auswertung kann dabei an beliebigen Positionen erfolgen, deren Menge nur von der Anzahl der Freiheitsgrade des Simulationsmodells beschränkt ist. Es können extreme, zerstörende Fälle analysiert werden. Dabei ist es unter anderem vorteilhaft, reine Signale ohne Rauschanteile zu verwenden. Simulationen von Triebwerksläufen sind verhältnismäßig kostengünstig.

Nachteilig wirkt es sich jedoch bei der Simulation aus, daß sie bestimmten Annahmen unterworfen ist, z.B. bei der Modellierung von Verbindungen und Dämpfungseigenschaften, usw.. Zu den weiteren Nachteilen der Simulation zählen deren begrenzte Gültigkeit, z.B. nur für ein bestimmtes Frequenzband (normalerweise der untere Frequenzbereich), und daß manche Effekte nur mit sehr hohem Aufwand berücksichtigt werden können. Darüber hinaus beschreiben die Simulationsmodelle nur bestimmte Eigenschaften der Struktur mit einer hohen Genauigkeit. Andere Eigenschaften, wie z.B. thermische Wirkungen, usw. werden dagegen nur global berücksichtigt.

Demgegenüber besitzt die Messung die folgenden Vorteile. Es wird die tatsächliche, gegenwärtige Struktur verwendet, keine physikalische Idealisierung derselben. Insbesondere bei den Entwicklungs- und Zertifizierungsprozessen werden bestimmte Lastfälle analysiert, welche bestimmten Fehlern entsprechen, z.B. Blattverlust an verschiedenen Stufen der einzelnen Komponenten des Triebwerks. Außerdem können bei Messungen zusätzlich Betriebsparameter berücksichtigt werden, insbesondere bei Untersuchungen im Flug werden eine Reihe von zusätzlichen Parametern aufgezeichnet.

Jedoch ergeben sich auch bei der Messung Probleme. Nachteilig wirkt sich die Ungenauigkeit bzw. Streuung der Messung aus, ebenso wie Meßfehler oder Rauscheffekte. Zusätzlich sind die Individualität der Triebwerke und die veränderlichen Bezugsbedingungen problematisch. Die Beobachtung kann nur an wenigen festen Positionen durchgeführt werden.

Daher ergeben sich die folgenden Erfordernisse zur Überwindung dieser Nachteile und Probleme:

Es muß eine extensive numerische Erzeugung von Schwingungskennungssignalen erfolgen, welche von einer Generierung experimenteller Signaturen begleitet werden soll. Dazu ist eine Definition von Parametern, die beobachtet und zur Diagnose verwendet werden sollen, ebenso wie eine Erstellung eines Fehlerkatalogs erforderlich. Die zu identifizierenden Fehler müssen definiert werden und eine Analyse von Verbindungen bzw. einer Beziehung zwischen Fehlern muß stattfinden.

Ebenso ist eine Extraktion von Merkmalen und eine Analyse von Verbindungen bzw. Beziehungen zwischen Symptomen, Begleiterscheinungen bzw. Anzeichen nötig. Parameter müssen identifiziert werden und Triebwerksmodelle entwickelt und Verbindungen bzw. Beziehungen zwischen Fehlern und Begleiterscheinungen bzw. Anzeichen erzeugt werden.

Weiterhin ist eine Entwicklung von umfassenden Diagnosesystemen auf der Grundlage von Neuronalen Netzen unter Berücksichtigung von verschiedenen physikalischen Informationen (Schwingungen, Leistungsmerkmalen, Temperaturen usw.) und statistischen bzw. probabilistischen Informationsquellen erforderlich.

Die Eigenschaften der Neuronalen Netzes, wie beispielsweise der Typ bzw. der Art, die Architektur, das Trainingsverfahren, usw. müssen definiert werden. Darüber hinaus sollen Untersuchungen über eine mögliche Anwendung von Neuronalen Netzen in Kombination mit Fuzzy-Logik erfolgen. Schließlich werden die Simulationsmodelle bzw. -verfahren und die Meßtechniken über Empfindlichkeitsund Korrelationsanalyse optimiert.

Die Hauptprobleme, die bei meßtechnischen Betrachtungen auftreten, sind die Datenstreuung, die Identifizierung von Rauschdaten, die begrenzten Datenmengen für eine vollständige Analyse und die sich verändernde Bezugsbedingung für jedes Triebwerk. Mögliche Problemlösungen sind eine Musterzuordnung, eine Klassifizierung und eine Identifizierung der Information mittels neuronaler bzw. neuro-fuzzy Methoden.

Erfindungsgemäß werden zur Diagnose von Triebwerkszuständen nicht, wie herkömmlich, nur die erfaßten Schwingungssignale des Triebwerks zur Diagnose verwendet, sondern auch andere Betriebsparameter, wie beispielsweise Höhe, Temperatur, usw., die den Zustand des Triebwerks ebenfalls mitbeeinflussen. Weiterhin sollen auch statistische und probabilistische Betrachtungen zusätzlich berücksichtigt werden.

Dabei erfolgt die Diagnose des Triebwerkszustands unter Verwendung eines lernenden, intelligenten Systems. Dieses System wird von der Entwicklungsphase bis zur Serienproduktion eingesetzt. Es werden zusätzliche physikalische Informationen, wie Betriebsparameter, Temperaturen, Leistungsparameter, usw. zur Diagnose verwendet. Der gegenwärtige Baustandard und die Vorgeschichte des Triebwerks so wie die Symptome und deren verifizierte Fehlerursachen werden in dem erfindungsgemäßen System und Verfahren systematisch aufgezeichnet und ausgewertet.

Insbesondere wird das intelligente System unter Verwendung physikalischer Simulationsmodelle trainiert, wobei die physikalischen Modelle unter Verwendung einer Korrelation mit den Messungen iterativ bzw. schrittweise verbessert werden. Zusätzlich wird das intelligente System mit Hilfe tatsächlicher bzw. realer Ereignisse und Vorfälle trainiert.

Außerdem werden die Fehlerfälle aller Kunden unter Verwendung einer gemeinsamen Datenbasis bzw. Datenbank von den Produzenten gesammelt und ausgewertet.

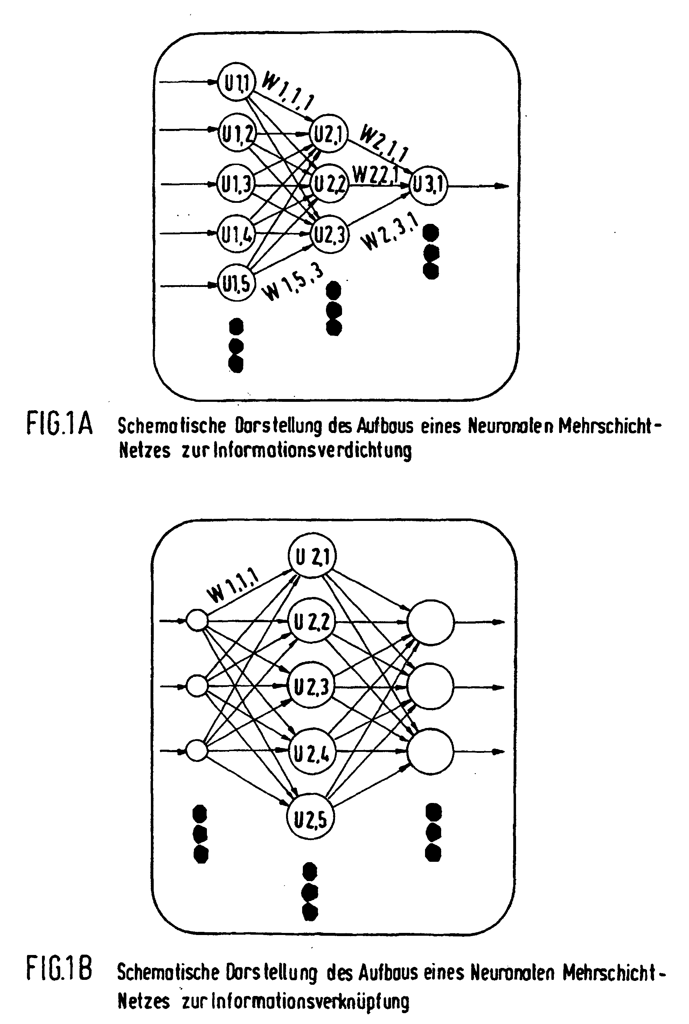

In einem derartigen intelligenten System werden Neuronale Netze verwendet. Ein Neuronales Netz besteht aus einer Vielzahl von Neuronen, von denen jedes eine nichtlineare Eingangs/Ausgangskennlinie besitzt und die durch Verbindungselemente mit jeweils wechselseitig unabhängigen Gewichtskoeffizienten miteinander verbunden sind. Die Gewichtskoeffizienten können durch einen Lemvorgang verändert werden. Dabei wird das Ausgangssignal des Neuronalen Netzes aufgrund einer besonderen Kombination von Eingangswerten mit einem bekannten Wert (Lehrwert) verglichen, der zu diesen Eingangswerte korrespondiert. Aus dieser Gegenüberstellung wird eine Modifikation der Werte der Gewichtskoeffizienten, wie beispielsweise um den Ausgangswert vom Neuronalen Netz näher zum Lehrwert zu bringen, abgeleitet. Dazu wird ein Lernalgorithmus bzw. - rechenverfahren verwendet. Der Lemvorgang wird aufeinanderfolgend für eine Anzahl von verschiedenen Lehrwerten und entsprechenden Eingangswertkombinationen wiederholt. Dies gilt insbesondere für das Neuronale Netz gemäß Fig. 1B, bei dessen Training z.B. die Methode der überwachten "Back Propagation" verwendet werden kann. Weiterhin besteht auch die Möglichkeit andere Methoden für das Training der Neuronalen Netze einzusetzen. Dabei handelt es sich beispielsweise um nicht überwachte Verfahren (z.B. Methode der selbstorganisierenden Karten von Kohonen), welche insbesondere für Klassifikationsaufgaben wie im Neuronalen Netz von Fig. 1A verwendet werden.

Ein Neuronales Mehrschicht-Netz gemäß Fig. 1A und Fig. 1B ist aus aufeinanderfolgenden Schichten von Neuronen gebildet, mit Zwischenverbindungen durch Verbindungselemente, die zwischen den Neuronen einer Schicht und Neuronen von vorhergehenden und nachfolgenden Schichten verbunden sind. Die Verbindungselemente multiplizieren die Ausgangssignale mit Gewichtskoeffizienten W_{n,i,j} bzw. W_{n,j,k}. Während des Lemvorgangs eines Neuronalen Netzes sind diese Gewichtskoeffizienten W_{n,i,j} bzw. W_{n,j,k} veränderlich und werden wechselseitig unabhängig bestimmt. Die Werte der Gewichtskoeffizienten W_{n,i,j}, welche die Eingangs- mit der Zwischenschicht verbinden, können als die jeweiligen Kopplungsstärken zwischen Neuronen der Zwischenschicht U_{2,j} und den Neuronen der Eingangsschicht U_{1,i} betrachtet werden. Wenn die Ausgangsschicht U_{3,k} des Neuronalen Netzes nur aus einem einzelnen Neuron U_{3,1} besteht, wird ein einzelner Ausgangswert von dem letzten Neuron des Neuronalen Netzes ansprechend auf eine bestimmte Kombination von der Eingangsschicht des Neuronalen Netzes zugeführten Eingangssignalwerten erzeugt.

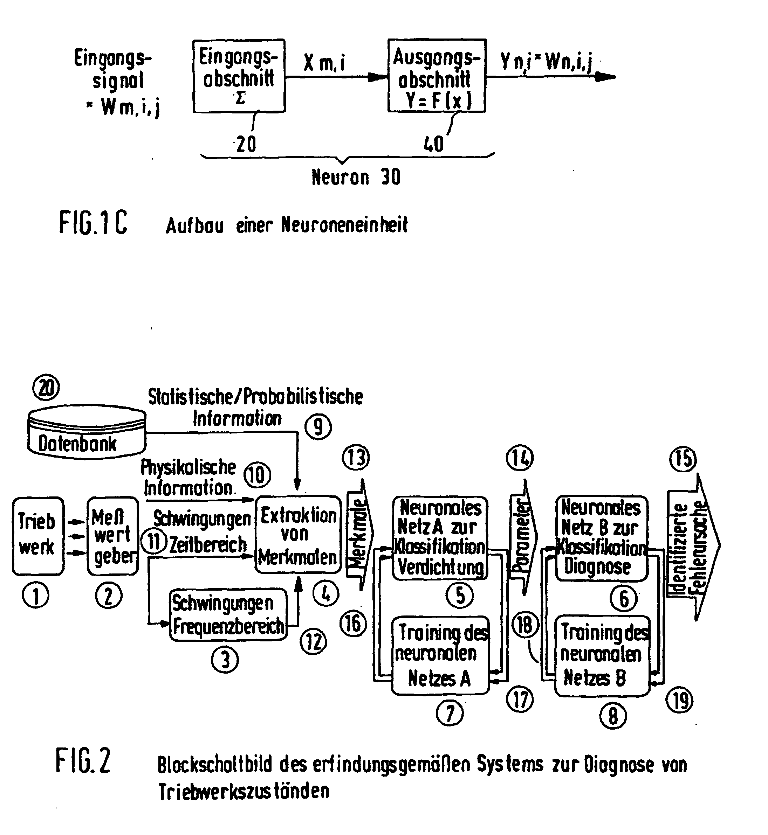

Fig. 1C zeigt ein Neuron 30, das als aus einem Eingangsabschnitt 20 und einem Ausgangsabschnitt 40 bestehend betrachtet werden kann. Der Eingangsabschnitt 20 summiert die ihm zugeführten gewichteten Eingangssignalwerte auf, wobei jeder dieser Eingangssignalwerte mit einem entsprechenden Gewichtskoeffizienten W_{m,i,j} multipliziert wird. Das sich ergebende summierte Ausgangssignal, das vom Eingangsabschnitt 20 des Neurons 30 erzeugt wird, wird als X_{m,i} bezeichnet und dem Ausgangsabschnitt 40 des Neurons zugeführt. Der Ausgangsabschnitt 40 führt eine Verarbeitung entsprechend einer nicht-linearen Funktion Y=F(x) aus, um das Ausgangssignal Y_{n,i} zu erhalten, das einem oder mehreren Neuronen der nachfolgenden Schicht zugeführt wird, nachdem es mit jeweiligen Gewichtskoeffizienten multipliziert wurde.

Im folgenden wird nun schematisch das erfindungsgemäße System und Verfahren zur Diagnose von Triebwerkszuständen unter Bezugnahme auf Fig. 2 beschrieben.

Das erfindungsgemäße System und Verfahren erhält über Messwertgeber 2 physikalische Informationen 10, wie z.B. Druck und Temperatur in verschiedenen Ebenen eines Triebswerks 1 sowie auch Parameter aus der Gasverfolgung innerhalb des Triebwerks 1 und aus der Partikelanalyse in verbrauchtem Öl. Weiterhin erhält das System Schwingungsinformationen im Zeitbereich 11 und nach Verarbeitung derselben durch eine Schwingungsanalyseeinrichtung 3 Informationen im Frequenzbereich 12. Zusätzlich werden dem System Informationen übertragen, welche aus statistischen und probabilistischen Betrachtungen 9 aus Daten einer entsprechenden Datenbank 20 resultieren. Aus dieser Fülle von Informationen werden unter Anwendung spezieller Algorithmen von einem Modul zur Merkmalextraktion 4 Merkmale 13 extrahiert, die das Triebwerk umfassend charakterisieren.

Das erfindungsgemäße System bzw. Verfahren verwendet ein erstes Neuronales Netz 5, das in der Eingangsschicht mehr Neuronen als in der Ausgangsschicht aufweist. Die Aufgabe dieses Netzes ist, die zugeführten Merkmale 13 zu klassifizieren und vor allem Beziehungen und Abhängigkeiten zwischen den Merkmalen zu identifizieren. Es werden Gruppen von Merkmalen gebildet, welche durch ausgewählte "Vertreter", die hier genannten Parameter 14, im weiteren Prozess berücksichtigt werden. Somit wird eine Datenverdichtung durch Beseitigung von redundanten Informationen erreicht.

Das erste Neuronale Netz 5 wird unter Anwendung verschiedener Methoden durch eine erste Lehreinrichtung 7 trainiert. Es wird unter anderem die Methode der "Back Propagation" mit den Datensätzen 16 und 17 verwendet, aber auch die Methode der "Selbstorganisierenden Karten" eingesetzt.
Weiterhin ist im erfindungsgemäßen System und Verfahren ein zweites Neuronales Netz 6 vorgesehen. Eingangssignale des zweiten nachgeschalteten Neuronalen Netzes 6 sind die identifizierten Parameter 14. Die Aufgabe dieses zweiten Neuronalen Netzes ist die Klassifikation und Erkennung von Zusammenhängen zwischen den Parametern 14 und bestimmten Fehlerkonstellationen 15. Dieser Vorgang wird im Rahmen des erfindungsgemäßen Systems Diagnose genannt, da die Fehler 15 über die Parameter 14 und diese wiederum über die Merkmale 13 mit physikalisch interpretierbaren Eigenschaften des Treibwerks 1 kausal assoziiert werden.

Die verschiedenen Schichten des zweiten Neuronalen Netzes 6 bestehen aus jeweils mehreren Neuronen. Die Anzahl der verdeckten Schicht wird, wie üblich bei Klassifizierungsprozessen, relativ klein sein (1 oder 2 Schichten). Die Anzahl der Neuronen wird jedoch in der Regel größer als die der äußeren Schichten sein. Ausgangssignal des zweiten Neuronalen Netzes ist ein Diagnosesignal, welches auf eine bestimmte Fehlerkonstellation 15 hinweist.

Das Training des zweiten Neuronalen Netzes durch eine zweite Lehreinrichtung 8 wird mit Hilfe der überwachten "Back Propagation"-Methode durchgeführt. Dabei werden bekannte Fehler 19 und deren Symptome in Form von Parametern 18 eingesetzt.

Nun wird genauer darauf eingegangen, welche Eingangssignale dem Modul zur Merkmalextraktion 4 zugeführt werden. Wie vorstehend bereits erwähnt, sind diese Eingangsdaten Schwingungssignale im Zeit- und Frequenzbereich 11 bzw. 12 und zusätzlich physikalische 10 sowie statistisch/probabilistische 9 Beobachtungsparameter. Im Modul zur Merkmalsextraktion werden diese Informationen getrennt verarbeitet, es werden aber gemeinsame Kennungen für eine weitere Bearbeitung vorgesehen.

Zunächst zu den Schwingungssignalen im Zeitbereich 11. Hierbei werden Verfahren und Techniken verwendet, wie sie zur Spracherkennung üblich sind. Außerdem werden der Effektivwert (RMS = root mean Square), Hüllkurven, Modulationen, Absolutwerte, Leistungsanalyse, statistische Parameter (Standardabweichungen, usw.), Verteilungsfunktionen, Wavelet-Analyse, usw. der Schwingungssignale im Zeitbereich als Indikatoren verwendet.

Dagegen wird für die Schwingungssignale im Frequenzbereich 12 eine Darstellung in Form eines sogenannten Wasserfalldiagramms gewählt. Diese graphische Informationsdarstellung wird dann mit Bildverarbeitungsverfahren behandelt und daraus werden entsprechende Merkmale aussortiert. Somit wird eine globale Betrachtung realisiert, da alle Bereiche des Bildes (Wasserfalldiagramm) mit der gleichen Gewichtung verarbeitet werden. Weiterhin werden geometrische Betrachtungen durchgeführt, um Indikatoren, wie beispielsweise den Schwerpunkt des gesamten Bildes oder Schwerpunkte bestimmter Bildregionen, welche nach bestimmten physikalischen Überlegungen definiert werden (z.B. subharmonischer oder superharmonischer Bereich), zu erzeugen. Die sogenannten "Sky-Lines" des Wasserfallsdiagramms, betrachtet aus der Perspektive der Frequenz- bzw. der Zeit/Drehzahlachse, liefern zusätzliche Bildmerkmale.

Außerdem wird die Information der Wasserfalldiagramme numerisch erfasst. Somit ergibt sich zusätzlich die Möglichkeit, Methoden aus der Matrizen- und Vektorrechnung (verschiedene Normen, Längen, usw.), wie beispielsweise Ermittlung von maximalen Werten, Mittelwerten, Summennormen, Euklidischen Normen, Korrelationskoeffizienten, Regressionskoeffizienten, Standardabweichungen, usw. zur Gewinnung von Indikatoren einzusetzen. Weiterhin werden aus der Entwicklung der Amplituden der zu der Betriebsdrehzahl des jeweiligen Rotors, deren Mehrfachen und Kombinationen zugeordneten Schwingungen Informationen extrahiert, welche die Erzeugung zusätzlicher Merkmale erlauben. Eine andere Alternative der Bearbeitung der numerischen Information ist die Anwendung von Verfahren zur Systemidentifikation (direct estimation method usw.) im Frequenzbereich bezogen auf einzelne Spektra (d.h. quasi-konstante Drehzahl) und/oder auf die oben genannten Verläufe der Drehzahlharmonischen. Die Berücksichtigung von Übertragungsfunktionen sowie eine Verteilungsanalyse der numerischen Daten liefern zusätzliche Indikatoren aus den Schwingungssignalen im Frequenzbereich.

Eine vollständig andere Quelle von Merkmalen sind Beobachtungen von zusätzliche physikalischen Parametern 10. Zu dieser Gruppe von Parametern zählen der Ölverbrauch bei bestimmten Triebwerksläufen, Leistungsbezugszahlen wie Druck und Temperatur in bestimmten Triebwerksebenen, Partikelanalyse im verbrauchten Öl und in den Triebwerksabgasen sowie die Gasweganalyse. Eine andere Alternative ergibt sich aus der statistischen bzw. probabilistischen Betrachtung der Fehler 9. Mit Hilfe dieser Analyse können bestimmte Triebwerkskomponenten oder -bauteile als besonders anfällig eingestuft werden. Diese Information wird in Form von Merkmalen verwendet.

Die Merkmale 13 resultierend aus dem entsprechenden Modul zur Merkmalextraktion 4 sind dann die Eingangsdaten der Eingangsschicht des ersten Neuronalen Netzes 5. Die Aufgabe dieses Netzes ist die Komprimierung der durchaus umfangreichen Eingangsinformationen und die Generierung von weitgehend unabhängigen Parametern 14.

Dem zweiten Neuronalen Netz 6 werden die vom ersten Neuronalen Netz 5 ausgegebenen Parameter 14 zugeführt und dieses gibt daraufhin ein entsprechendes Diagnosesignal (Fehlersignal) 15 aus.

Somit kann durch die Verwendung der zwei Neuronalen Netze mit dem erfindungsgemäßen System bzw. Verfahren eine zuverlässige Diagnose des Triebwerkszustands erreicht werden.

Anstelle der beiden Neuronalen Netze könnten auch Neuronale Netze in Verbindung mit Fuzzy-Logik oder reine Fuzzy-Logik-Schaltungen verwendet werden.

## Patentansprüche

1. System zur Diagnose von Triebwerkszuständen, mit
einer Einrichtung zur Zuführung statistisch/probabilistischer Informationen (9) über die Fehlerquote einzelner Triebwerkskomponenten resultierend aus einer Bewertung einer entsprechenden Datenbank (20) und/oder
einer Vielzahl von Messwertgebern (2) zur Erfassung physikalischer Informationen (10), wie beispielsweise Drücke und Temperaturen in verschiedenen Triebwerksebenen und außerdem Parameter aus einer Partikelanalyse in verbrauchtem Öl und in Triebwerksabgasen sowie Parameter aus einer Gasweganalyse, und
einer Vielzahl von Messwertgebern (2) zur Erfassung von Schwingungsinformationen im Zeitbereich (11) von einem Triebwerk (1),
einer Schwingungsanalyseeinrichtung (3) zur Erzeugung von Schwingungsinformationen im Frequenzbereich (12) aus den Schwingungsinformationen im Zeitbereich (11),
einem Modul zur Merkmalextraktion (4) zur Verarbeitung der physikalischen Informationen (10) und/oder der statistisch/probabilistischen Informationen (9) und der Schwingungsinformationen im Zeit- und Frequenzbereich (11, 12) und zur Extraktion einer Reihe von Merkmalen (13), die den Triebwerkszustand umfassend beschreiben,
einem ersten Neuronalen Netz (5), an das die Merkmale (13) angelegt sind, zur Klassifizierung der Merkmale (13), zur Identifizierung von Beziehungen und Abhängigkeiten zwischen Merkmalen und zur dementsprechenden
Durchführung einer Informationsverdichtung und zur Ausgabe von Parametern (14), wobei das erste Neuronale Netz (5) eine Eingangsschicht, eine oder mehrere Zwischenschichten und eine Ausgangsschicht aus Neuronen aufweist, wobei die Eingangsschicht mehr Neuronen als die Zwischenschicht(en) und diese wiederum mehr Neuronen als die Ausgangsschicht aufweist und die Neuronen einer Schicht über eine Vielzahl von Verbindungselementen mit variablen Gewichtskoeffizienten verbunden sind,
einer ersten Lehreinrichtung (7) zur Zuführung von Lerneingangssignalen (16) zum ersten Neuronalen Netz (5) und zum Vergleich des daraufhin vom ersten Neuronalen Netz (5) ausgegebenen Ausgangssignals (14) mit einem Lehreingangssignal (17) und zur Änderung von variablen Gewichtskoeffizienten des ersten Neuronalen Netzes (5) mittels Anwendung eines vorbestimmten Lernalgorithmus entsprechend den Differenzen zwischen dem Lehreingangssignal (17) und dem Ausgangsignal (14) oder zur Realisierung eines nicht überwachten Trainings des ersten Neuronalen Netzes (5) mit Hilfe der Lerneingangssignale (16) allein,
einem zweiten Neuronalen Netz (6), an das die durch das ersten Neuronale Netz (5) ausgegebenen Parameter (14) angelegt sind, zur Klassifizierung der Parameter (14), zur Erkennung von Zusammenhängen zwischen den Parametern (14) und bestimmten Fehlerkonstellationen, zur dementsprechenden Durchführung einer Informationsverknüpfung und zur Ausgabe eines Diagnosesignals (15), wobei das zweite Neuronale Netz (6) eine Eingangsschicht, eine oder mehrere Zwischenschichten und eine Ausgangsschicht aus Neuronen aufweist, wobei die Eingangs- und die Ausgangsschicht weniger Neuronen als die Zwischenschicht(en) aufweisen, und die Neuronen einer Schicht mit den Neuronen der ihr nachfolgenden Schicht über eine Vielzahl von Verbindungselementen mit variablen Gewichtskoeffizienten verbunden sind, und
einer zweiten Lehreinrichtung (8) zur Zuführung von Lerneingangssignalen (18) zum zweiten Neuronalen Netz (6) und zum Vergleich des daraufhin vom zweiten Neuronalen Netz (6) erhaltenen Ausgangssignals (15) mit einem Lehreingangssignal (19) und zur Änderung von variablen Gewichtskoeffizienten des zweiten Neuronalen Netzes (6) mittels Anwendung eines vorbestimmten Lernalgorithmus entsprechend den Differenzen zwischen dem
Lehreingangssignal (19) und dem Ausgangssignal (15).

2. System nach Anspruch 1, wobei das Modul zu Merkmalextraktion (4) physikalische Parameter (10), wie Ölverbrauch bei bestimmten Triebwerksläufen, Leistungsbezugszahlen wie Druck und Temperatur in bestimmten Triebwerksebene, Parameter aus einer Partikelanalyse in verbrauchtem Öl und in Triebwerksabgasen sowie Parameter aus einer Gasweganalyse, verwendet.

3. System nach Anspruch 1, wobei das Modul zur Merkmalextraktion (4) Verfahren verwendet, wie sie zur Spracherkennung üblich sind, und unter anderem Effektivwerte, Eigenschaften der Hüllkurven, Modulationen, Absolutwerte, Leistungsanalysen, statistische Parameter, Verteilungsfunktionen und eine Wavelet-Analyse der Schwingungsinformationen im Zeitbereich (11) als Merkmale extrahiert.

4. System nach Anspruch 1, wobei die Schwingungsanalyseeinrichtung (3) die Schwingungssignale im Zeitbereich behandelt und daraus entsprechende Schwingungsinformationen im Frequenzbereich (12) ermittelt.

5. System nach einem der Ansprüche 1 oder 4, wobei das Modul zur Merkmalextraktion (4) eine Informationsdarstellung in Form eines sogenannten Wasserfalldiagramms verwendet, diese Informationsdarstellung mit Bildbearbeitungsverfahren behandelt und daraus entsprechende Merkmale aus den Schwingungsinformationen im Frequenzbereich (12) ermittelt.

6. System nach Anspruch 5, wobei das Modul zur Merkmalextraktion (4) außerdem geometrische Betrachtungen des gesamten Bildes oder bestimmter Bildregionen durchführt und /oder das Modul zur Merkmalextraktion (4) außerdem sogenannten "Sky-Lines" des Wasserfalldiagramms aus der Perspektive der Frequenz- b m . der Zeit/Drehzahlachse betrachtet.

7. System nach Anspruch 5, wobei das Modul zur Merkmalextraktion (4) die Schwingungsinformation der Wasserfalldiagramme außerdem numerisch erfasst und Methoden aus der Matrizen- und Vektorrechnung oder Verfahren zur Systemidentifikation im Frequenzbereich zur Gewinnung von Merkmalen aus den Schwingungsinformationen im Frequenzbereich (12) und/oder Übertragungsfunktionen sowie eine Verteilungsanalyse der numerischen Daten einsetzt.

8. Verfahren zur Diagnose von Triebwerkszuständen, mit den Schritten:
Zuführen statistisch/probabilistischer Informationen (9) über die Fehlerquote einzelner Triebwerkskomponenten resultierend aus einer Bewertung einer entsprechenden Datenbank (20) und/oder
Erfassen physikalischer Informationen (10), wie beispielsweise Drücke und Temperaturen in verschiedenen Triebwerksebenen, durch eine Vielzahl von Messwertgebern (2), außerdem Parameter aus einer Partikelanalyse in verbrauchtem Öl und in Triebwerksabgasen sowie Parameter aus einer Gasweganalyse, und/oder
Erfassen von Schwingungsinformationen im Zeitbereich (11) von einem Triebwerk (1) durch eine Vielzahl von Messwertgebern (2) und
Erzeugen von Schwingungsinformationen im Frequenzbereich (12) aus den Schwingungsinformationen im Zeitbereich (11) durch eine Schwingungsanalyseeinrichtung (3),
Verarbeiten der physikalischen Informationen (10) und/oder der statistisch-/probabilistischen Informationen (9) und/oder der Schwingungsinformationen im Zeit- und Frequenzbereich (11, 12) und Extrahieren einer Reihe von Merkmalen (13), die den Triebwerkszustand umfassend beschreiben, durch ein Modul zur Merkmalextraktion (4),
Klassifizieren der Merkmale (13), Identifizieren von Beziehungen und Abhängigkeiten zwischen Merkmalen und dementsprechend Durchführen einer Informationsverdichtung und Ausgeben von Parametern (14) durch ein erstes Neuronales Netz (5), an das die Merkmale (13) angelegt werden, wobei das erste Neuronale Netz (5) eine Eingangsschicht, eine oder mehrere Zwischenschichten und eine Ausgangsschicht aus Neuronen aufweist, wobei die Eingangsschicht mehr Neuronen als die Zwischenschicht(en) und diese wiederum mehr Neuronen als die Ausgangsschicht aufweist und die Neuronen einer Schicht über eine Vielzahl von Verbindungselementen mit variablen Gewichtskoeffizienten verbunden sind,
Zuführen von Lerneingangssignalen (16) zum ersten Neuronalen Netz (5) und Vergleichen des daraufhin vom ersten Neuronalen Netz ( 5 ) ausgegebenen Ausgangssignals (14) mit einem Lehreingangssignal (17) und Ändern von variablen Gewichtskoeffizienten des ersten Neuronalen Netzes (5) mittels Anwendung eines vorbestimmten Lernalgorithmus entsprechend den Differenzen zwischen dem Lehreingangssignal (17) und dem Ausgangssignal (14) oder zur Realisierung eines nicht überwachten Trainings des ersten Neuronalen Netzes ( 5 ) mit Hilfe der Lerneingangssignate (16) allein durch eine erste Lehreinrichtung (7),
Klassifizieren der Parameter (14), Erkennen von Zusammenhängen zwischen den Parametern (14) und bestimmten Fehlerkonstellationen, dementsprechendes Durchführen einer Informationsverknüpfung und Ausgeben eines Diagnosesignals (15) durch ein zweites Neuronalen Netz (6), an das die durch das ersten Neuronale Netz (5) ausgegebenen Parameter (14) angelegt werden, wobei das zweite Neuronale Netz (6) eine Eingangsschicht, eine oder mehrere Zwischenschichten und eine Ausgangsschicht aus Neuronen aufweist, wobei die Eingangs- und die Ausgangsschicht weniger Neuronen als die Zwischenschicht(en) aufweisen, und die Neuronen einer Schicht mit den Neuronen der ihr nachfolgenden Schicht über eine Vielzahl von Verbindungselementen mit variablen Gewichtskoeffizienten verbunden sind, und
Zuführen von Lerneingangssignalen (18) zum zweiten Neuronalen Netz (6) und Vergleichen des daraufhin vom zweiten Neuronalen Netzes (6) erhaltenen Ausgangssignals (15) mit einem Lehreingangssignal (19) und Ändern von variablen Gewichtskoeffizienten des zweiten Netzes (6) mittels Anwendung eines vorbestimmten Lernalgorithmus entsprechend den Differenzen zwischen dem Lehreingangssignal (19) und dem Ausgangssignal (15) durch eine zweite Lehreinrichtung (8).

9. Verfahren nach Anspruch 8, wobei die erfassten physikalischen Parameter (10) einen Ölverbrauch bei bestimmten Triebwerksläufen, Leistungsbezugszahlen wie Druck und Temperatur in bestimmten Triebwerksebenen, Parameter aus einer Partikelanalyse in verbrauchtem Öl und in Triebwerksabgasen sowie Parameter aus einer Gasweganalyse enthalten.

10. Verfahren nach Anspruch 8, wobei bei der Merkmalextraktion anhand der statistischen/probalistischen Informationen (9) bestimmte Triebwerkskomponenten oder -bauteile beispielsweise als besonders anfällig eingestuft werden und diese Informationen in Form von Merkmalen (13) ausgegeben werden.

11. Verfahren nach Anspruch 8, wobei bei dem Verarbeiten der Informationen und Extrahieren von Merkmalen Verfahren verwendet, wie sie zur Spracherkennung üblich sind, und untern anderem Effektivwerte, Eigenschaften der Hüllkurven, Modulationen, Absolutwerte, Leistungsanalysen, statistische Parameter, Verteilungsfunktionen und eine Wavelet-Analyse der Schwingungsinformationen im Zeitbereich (11) als Merkmale extrahiert werden.

12. Verfahren nach Anspruch 8, wobei die Schwingungsinformation im Zeitbereich (11) mit einer Schwingungsanalyseeinrichtung (3) bearbeitet wird und daraus entsprechende Schwingungsinformationen im Frequenzbereich (12) ermittelt werden.

13. Verfahren nach einem der Ansprüche 8 oder 12, wobei beim Verarbeiten von Schwingungsinformationen im Frequenzbereich (12) eine Informationsdarstellung in Form eines sogenannten Wasserfalldiagramms verwendet wird, diese Informationsdarstellung mit Bildbearbeitungsverfahren behandelt wird und daraus entsprechende Merkmale aus den Schwingungsinformationen im Frequenzbereich (12) ermittelt werden.

14. Verfahren nach Anspruch 13, wobei beim Verarbeiten von Schwingungsinformationen im Frequenzbereich (12) außerdem geometrische Betrachtungen des gesamten Bilds oder bestimmter Bildregionen durchführt werden und/oder beim Bearbeiten von Schwingungsinformationen im Frequenzbereich (12) außerdem sogenannten "Sky-Lines" des Wasserfalldiagramms aus der Perspektive der Frequenz- bzw. der Zeit/Drehzahlachse betrachtet und daraus entsprechenden Merkmale extrahiert werden.

15. Verfahren nach Anspruch 13, wobei beim Bearbeiten von Schwingungsinformationen im Frequenzbereich (12) die Information der Wasserfalldiagramme außerdem numerisch erfasst werden und Methoden aus der Matrizen- und Vektorrechnung oder Verfahren zur Systemidentifikation im Frequenzbereich zur Gewinnung von Schwingungsinformationen im Frequenzbereich (12) und/oder Übertragungsfunktionen sowie eine Verteilungsanalyse der numerischen Daten eingesetzt werden.

## Claims

1. System for the diagnosis of engine conditions, with
a device for the provision of statistical / probabilistic information (9) about the proportion of defective individual engine components resulting from an evaluation of a corresponding databank (20) and/or
a plurality of measurement value emitters (2) for the collection of physical information (10) such as pressures and temperatures at various engine levels and in addition a particle analysis in used oil and in engine exhaust gases, and parameters from a gas path analysis, and
a plurality of measurement value emitters (2) for the collection of vibration information in the time range (11) of an engine (1),
a vibration analysis device (3) for the production of vibration information in the frequency range (12) from the vibration information in the time range (11),
a module for the extraction of characteristics (4) for the processing of the physical information (10) and/or the statistical / probabilistic information (9) and the vibration information in the time and frequency ranges (11, 12) and for the extraction of a series of characteristics (13) that describe comprehensively the condition of the engine,
a first neuronal network (5) to which the characteristics (13) are supplied, for the classification of the characteristics (13), for the identification of relationships and dependences between characteristics and for the corresponding
implementation of information compression and the output of parameters (14), the first neuronal network (5) comprising an input layer, one or more intermediate layers and an output layer of neurones, the input layer having more neurones than the intermediate layer(s) and this/these in turn more neurones than the output layer, the neurones of any layer being connected via a plurality of connection elements with various weighting coefficients,
a first teaching device (7) for the provision of teaching input signals (16) to the first neuronal network (5) and for comparing the output signal (14) emitted from the first neuronal network (5) with a teaching input signal (17) and for the modification of variable weighting coefficients of the first neuronal network (5) by applying a predetermined teaching algorithm in accordance with the differences between the teaching input signal (17) and the output signal (14) or for implementing an unsupervised training of the first neuronal network (5) with the aid of the teaching input signals (16) alone,
a second neuronal network (6) to which the parameters (14) emitted by the first neuronal network (5) are supplied, for the classification of the parameters (14), for the recognition of relationships between the parameters (14) and certain defect constellations, for correspondingly creating information links and for the output of a diagnosis signal (15), the second neuronal network (6) comprising an input layer, one or more intermediate layers and an output layer of neurones, such that the input and output layers have fewer neurones than the intermediate layer(s) and the neurones of one layer are connected to the neurones of the next layer via a plurality of connection elements with variable weighting coefficients, and
a second teaching device (8) for the provision of teaching input signals (18) to the second neuronal network (6) and for comparing the output signal (15) then obtained from the second neuronal network (6) with a teaching input signal (19) and for the modification of variable weighting coefficients of the second neuronal network (6) by applying a predetermined teaching algorithm in accordance with the differences between the
teaching input signal (19) and the output signal (15).

2. System according to Claim 1, such that the module for the extraction of characteristics (4) makes use of physical parameters (10) such as oil consumption in particular engine running conditions, performance-related figures such as pressure and temperature at certain engine levels, parameters from a particle analysis in used oil and in engine exhaust gases and parameters from a gas path analysis.

3. System according to Claim 1, such that the module for the extraction of characteristics (4) makes use of methods of the type usual for speech recognition and, among other things, extracts effective values, properties of the envelope curves, modulations, absolute values, performance analyses, statistical parameters, distribution functions and a wavelet analysis of the vibration information in the time range (11) as characteristics.

4. System according to Claim 1, such that the vibration analysis device (3) processes the vibration signals in the time range and determines therefrom corresponding vibration information in the frequency range (12).

5. System according to either of Claim s 1 or 4, such that the module for the extraction of characteristics (4) makes use of an information representation in the form of a so-termed waterfall diagram, processes this information representation by image enhancement techniques, and determines therefrom corresponding characteristics from the vibration information in the frequency range (12).

6. System according to Claim 5, such that the module for the extraction of characteristics (4) also takes into account geometrical features of the overall image or of particular image areas and/or the module for the extraction of characteristics (4) also takes into account so-termed "sky-lines" of the waterfall diagram from the perspective of the frequency or the time/rotation-speed axis.

7. System according to Claim 5, such that the module for the extraction of characteristics (4) also collects the vibration information of the waterfall diagram in numerical form and uses methods of matrix and vector computation or processes for system identification in the frequency range to obtain characteristics from the vibration information in the frequency range (12) and/or transfer functions and a distribution analysis of the numerical data.

8. Method for the diagnosis of engine conditions, comprising as steps:
the provision of statistical / probabilistic information (9) about the proportion of defective individual engine components resulting from an evaluation of a corresponding databank (20) and/or
the collection of physical information (10) such as pressures and temperatures at various engine levels, by a plurality of measurement value emitters (2), and in addition from a particle analysis in used oil and in engine exhaust gases, and parameters from a gas path analysis, and/or
the collection of vibration information in the time range (11) of an engine (1) by a plurality of measurement value emitters (2), and
the production of vibration information in the frequency range (12) from the vibration information in the time range (11) by a vibration analysis device (3),
processing of the physical information (10) and/or the statistical /probabilistic information (9) and/or the vibration information in the time and frequency ranges (11, 12) and extraction of a series of characteristics (13) that describe comprehensively the condition of the engine, by a module for the extraction of characteristics (4),
classification of the characteristics (13), identification of relationships and dependences between characteristics and the corresponding implementation of information compression and output of parameters (14) by a first neuronal network (5) to which the characteristics (13) are supplied, the first neuronal network (5) comprising an input layer, one or more intermediate layers and an output layer of neurones, the input layer having more neurones than the intermediate layer(s) and this/these in turn more neurones than the output layer, the neurones of any layer being connected via a plurality of connection elements with various weighting coefficients,
the provision of teaching input signals (16) to the first neuronal network (5) and comparison of the output signal (14) emitted from the first neuronal network (5) with a teaching input signal (17) and modification of variable weighting coefficients of the first neuronal network (5) by applying a predetermined teaching algorithm in accordance with the differences between the teaching input signal (17) and the output signal (14) or for the implementation of an unsupervised training of the first neuronal network (5) with the aid of the teaching input signals (16) alone, by a first teaching device (7)
classification of the parameters (14), recognition of relationships between the parameters (14) and certain defect constellations, the corresponding creation of information links and the output of a diagnosis signal (15) by a second neuronal network (6) to which the parameters (14) emitted by the first neuronal network (5) are supplied, the second neuronal network (6) comprising an input layer, one or more intermediate layers and an output layer of neurones, such that the input and output layers have fewer neurones than the intermediate layer(s) and the neurones of one layer are connected to the neurones of the next layer via a plurality of connection elements with variable weighting coefficients, and
the provision of teaching input signals (18) to the second neuronal network (6) and comparison of the output signal (15) then obtained from the second neuronal network (6) with a teaching input signal (19) and modification of variable weighting coefficients of the second neuronal network (6) by applying a predetermined teaching algorithm in accordance with the differences between the teaching input signal (19) and the output signal (15) by a second teaching device (8).

9. Method according to Claim 8, such that the physical parameters (10) collected include oil consumption in particular engine running conditions, performance-related figures such as pressure and temperature at certain engine levels, parameters from a particle analysis in used oil and in engine exhaust gases and parameters from a gas path analysis.

10. Method according to Claim 8, such that for the extraction of characteristics, on the basis of the statistical / probabilistic information (9) certain engine components or parts are for example classified as particularly vulnerable and this information is emitted in the form of characteristics (13).

11. Method according to Claim 8, such that in processing the information and extracting characteristics, methods are used of the type usual for speech recognition and, among other things, effective values, properties of the envelope curves, modulations, absolute values, performance analyses, statistical parameters, distribution functions and a wavelet analysis of the vibration information in the time range (11) are extracted as characteristics.

12. Method according to Claim 8, such that the vibration information in the time range (11) is processed by a vibration analysis device (3) and corresponding vibration information in the frequency range (12) is determined therefrom.

13. Method according to either of Claims 8 or 12, such that for the processing of vibration information in the frequency range (12), an information representation in the form of a so-termed waterfall diagram is used, this information representation is processed by image enhancement techniques, and corresponding characteristics from the vibration information in the frequency range (12) are determined therefrom.

14. Method according to Claim 13, such that in the processing of vibration information in the frequency range (12) geometrical features of the overall image or of particular image areas are also taken into account, and/or for the processing of vibration information in the frequency range (12) so-termed "sky-lines" of the waterfall diagram are also taken into account from the perspective of the frequency or the time/rotation-speed axis, and corresponding characteristics are extracted therefrom.

15. Method according to Claim 13, such that in the processing of vibration information in the frequency range (12) information of the waterfall diagram is also collected in numerical form, and methods of matrix and vector computation or processes for system identification in the frequency range and/or transfer functions and a distribution analysis of the numerical data are used to obtain vibration information in the frequency range (12).

## Revendications

1. Système de diagnostic d'états de groupes motopropulseurs, avec
un dispositif pour la délivrance d'information concernant des statistiques / des probabilités (9) sur le quota de pannes de composantes individuelles du groupe motopropulseur résultant d'une évaluation d'une banque de données correspondante (20) et/ou
une pluralité de capteurs de mesure (2) destinés à détecter des informations physiques (10), comme par exemple des pressions et des températures dans différents plans du groupe motopropulseur et par ailleurs des paramètres issus d'une analyse de particules dans de l'huile usagée et dans des gaz émanant du groupe motopropulseur, ainsi que des paramètres résultant d'une analyse du trajet du gaz et
une pluralité de capteurs de mesure (2) destinés à détecter des informations concernant les vibrations dans la plage de temporisation (11) d'un groupe motopropulseur (1),
un dispositif d'analyse des vibrations (3) destiné à générer des informations concernant les vibrations dans la plage de fréquences (12), à partir des informations concernant les vibrations dans la plage de temporisation (11),
un module pour l'extraction des caractéristiques (4) destiné à traiter les informations physiques (10) et/ou les informations concernant des statistiques /des probabilités (9) et les informations concernant les vibrations dans la plage de temporisation et de fréquences (11,12) et à extraire une série de caractéristiques (13), qui décrivent de façon globale l'état du groupe motopropulseur,
un premier réseau neuronal (5) sur lequel sont appliquées les caractéristiques (13), destiné à classifier les caractéristiques (13), à identifier les relations et les corrélations entre des caractéristiques et
destiné à procéder en conséquence à une compression des informations et à éditer des paramètres (14), le premier réseau neuronal (5) présentant une couche d'entrée, une ou plusieurs couches intermédiaires et une couche de sortie constituées de neurones, la couche d'entrée possédant plus de neurones que la (les) couche(s) intermédiaire(s), cette dernière possédant quant à elle plus de neurones que la couche de sortie et les neurones d'une couche étant reliés à des coefficients de poids variables, par l'intermédiaire d'une pluralité d'éléments de liaison,
un premier dispositif d'apprentissage (7) destiné à fournir des signaux d'apprentissage d'entrée (16) au premier réseau neuronal (5) et à comparer le signal de sortie (14) délivré ensuite par le premier réseau neuronal (5) par application d'un algorithme d'apprentissage prédéfini en fonction des différences entre le signal d'apprentissage d'entrée (17) et le signal de sortie (14) ou à procéder à un entraînement non surveillé du premier réseau neuronal (5), à l'aide du seul signal d'apprentissage d'entrée (16),
un second réseau neuronal (6), sur lequel sont appliqués les paramètres (14) édités par le premier réseau neuronal (5), destiné à classifier les paramètres (14), à identifier les corrélations entre les paramètres (14) et des constellations de pannes précises, pour procéder en conséquence à un chaînage d'informations et pour éditer un signal de diagnostic (15), le second réseau neuronal (6) présentant une couche d'entrée, une ou plusieurs couches intermédiaires et une couche de sortie constituées de neurones, la couche d'entrée et la couche de sortie comprenant moins de neurones que la (les) couche(s) intermédiaire(s) et les neurones d'une couche étant reliés aux neurones de la couche suivante par l'intermédiaire d'une pluralité d'éléments de liaison avec des coefficients de poids variables, et
un second dispositif d'apprentissage (8), destiné à fournir des signaux d'apprentissage d'entrée (18) au second réseau neuronal (6) et à comparer le signal de sortie reçu ensuite par le second réseau neuronal (6) avec un signal d'apprentissage d'entrée (19) et à modifier des coefficients de poids variables du second réseau neuronal (6), par application d'un algorithme d'apprentissage prédéfini, en fonction des différences entre le signal d'apprentissage d'entrée (19) et le signal d'apprentissage de sortie (15).

2. Système selon la revendication 1, où le module destiné à extraire des caractéristiques (4) utilise des paramètres physiques (10) comme la consommation d'huile à certains régimes du groupe motopropulseur, des chiffres de références en matière de puissance, comme la pression et la température dans des plans définis du groupe motopropulseur, des paramètres issus d'une analyse des particules dans l'huile usagée et dans les gaz d'échappement du groupe motopropulseur, ainsi que des paramètres émanant d'une analyse du trajet des gaz.

3. Système selon la revendication 1, où le module destiné à extraire des caractéristiques (4) utilise des procédés, qui sont usuels pour la reconnaissance vocale, et extrait entre autres en tant que caractéristiques des valeurs effectives, des caractéristiques de l'enveloppante, des modulations, des valeurs absolues, des analyses de puissance, des paramètres statistiques, des fonctions de répartition et une analyse d'ondelettes des informations concernant les vibrations dans la plage de temporisation (11).

4. Système selon la revendication 1, où le dispositif d'analyse des vibrations (3) traite les signaux de vibrations dans la plage de temporisation et détermine à partir de ces derniers des informations correspondantes concernant les vibrations dans la plage de temporisation (12).

5. Système selon l'une des revendications 1 ou 4, où le module destiné à extraire des caractéristiques (4) utilise une représentation des informations sous la forme d'un dénommé diagramme en cascade, traite cette représentation des informations à l'aide de procédés de traitement des images et détermine à partir de ces dernières des caractéristiques correspondantes à partir des informations concernant les vibrations dans la plage de temporisation (12).

6. Système selon la revendication 5, où le module destiné à extraire des caractéristiques (4) procède par ailleurs à des observations géométriques de l'image globale ou de certaines régions de l'image et/ou le module destiné à extraire des caractéristiques (4) considère par ailleurs des dénommées « Sky-Lines » du diagramme en cascade à partir de la perspective de la fréquence ou de l'axe de temps/de vitesse de rotation.

7. Système selon la revendication 5, où le module destiné à extraire des caractéristiques (4) détecte par ailleurs de façon numérique les informations concernant les vibrations dans les diagrammes en cascade et met en oeuvre des méthodes issues du calcul des matrices ou des vecteurs ou des méthodes pour l'identification du système dans la plage de fréquences pour acquérir des caractéristiques à partir des informations concernant les vibrations dans la plage de fréquences (12) et/ou des fonctions de transmission, ainsi qu'une analyse de répartition des données numériques.

8. Procédé de diagnostic d'états de groupes motopropulseurs, comportant les étapes suivantes :
délivrance d'informations concernant les statistiques / les probabilités (9) sur le quota de pannes de composantes individuelles du groupe motopropulseur, résultant d'une évaluation d'une banque de données correspondante (20) et/ou
détection d'informations physiques (10), comme par exemple des pressions et des températures dans différents plans du groupe motopropulseur, par une pluralité de capteurs de mesures (2), par ailleurs de paramètres issus d'une analyse des particules dans de l'huile usagée et dans les gaz d'échappement du groupe motopropulseur, ainsi que de paramètres issus d'une analyse du trajet des gaz, et/ou
détection d'informations concernant les vibrations dans la plage de temporisation (11) d'un groupe motopropulseur (1) par une pluralité de capteurs de mesures (2),
génération d'informations concernant les vibrations dans la plage de fréquences (12), à partir des informations concernant les vibrations dans la plage de temporisation (11) par un dispositif d'analyse des vibrations (3),
traitement des informations physiques (10) et des informations concernant des statistiques / des probabilités (9) et/ou des informations concernant les vibrations dans la plage de temporisation et de fréquence (11, 12) et extraction d'une série de caractéristiques (13), qui décrivent globalement l'état groupe motopropulseur, par un module destiné à extraire les caractéristiques (4),
classification des caractéristiques (13), identification de relations et de dépendances entre des caractéristiques et en conséquence de quoi, réalisation d'une compression des informations et édition de paramètres (14) à travers une premier réseau neuronal (5), sur lequel les caractéristiques (13) sont appliquées, le premier réseau neuronal (5) présentant une couche d'entrée, une ou plusieurs couches intermédiaires et une couche de sortie constituées de neurones, la couche d'entrée présentant plus de neurones que la (les) couche(s) intermédiaire(s) et cette dernière présentant quant à elle plus de neurones que la couche de sortie et les neurones d'une couche étant reliés par une pluralité d'éléments de liaison à coefficients de poids variables,
délivrance de signaux d'apprentissage d'entrée (16) au premier réseau neuronal (5) et comparaison du signal de sortie (14) délivré ensuite par le premier réseau neuronal (5) avec un signal d'apprentissage d'entrée (17) et modification de coefficients de poids variables du premier réseau neuronal (5) par application d'un logarithme prédéfini en fonction des différences entre le signal d'apprentissage d'entrée (17) et le signal de sortie (14) ou pour la réalisation d'un entraînement non surveillé du premier réseau neuronal (5), à l'aide des signaux d'apprentissage d'entrée (16), uniquement par un premier dispositif d'apprentissage (7),
classification des paramètres (14), identification de corrélations entre les paramètres (14) et détermination d'un constellation de pannes, en fonction de quoi, réalisation de chaînages d'informations et édition d'un signal de diagnostic (15) par un second réseau neuronal (6) sur lequel est appliqué le paramètre (14) édité par le premier réseau neuronal (5), le second réseau neuronal (6) présentant une couche d'entrée, une ou plusieurs couche(s) intermédiaire(s) et une couche de sortie constituées de neurones, la couche d'entrée et la couche de sortie présentant moins de neurones que la (les) couche(s) intermédiaire(s) et les neurones d'une couche étant reliés aux neurones de la couche suivante par une pluralité d'éléments de liaison à coefficients de poids variables, et
délivrance de signaux d'apprentissage d'entrée (18) au second réseau neuronal (6) et comparaison du signal de sortie (15) reçu alors par le second réseau neuronal (6) avec un signal d'apprentissage d'entrée (19) et modification de coefficients de poids variables du second réseau (6) par application d'un logarithme prédéfini en fonction des différences entre le signal d'apprentissage d'entrée (19) et le signal de sortie (15) par un second dispositif d'apprentissage (8).

9. Procédé selon la revendication 8, où les paramètres physiques détectés (10) contiennent une consommation d'huile à certains régimes du groupe motopropulseur, des chiffres de références en matière de puissance, comme la pression et la température dans des plans définis du groupe motopropulseur, des paramètres issus d'une analyse des particules dans l'huile usagée et dans les gaz d'échappement du groupe motopropulseur, ainsi que des paramètres émanant d'une analyse du trajet des gaz.

10. Procédé selon la revendication 8, où lors de l'extraction de caractéristiques, sur la base des informations (9) concernant des statistiques / des probabilités, certaines composantes ou certains éléments du groupe motopropulseur sont classifiés par exemple comme étant particulièrement vulnérables et lesdites informations sont éditées sous la forme de caractéristiques (13).

11. Procédé selon la revendication 8, où lors du traitement des informations et de l'extraction de caractéristiques, des procédés sont utilisés, qui sont usuels pour la reconnaissance vocale, et entre autres des valeurs effectives, des caractéristiques de l'enveloppante, des modulations, des valeurs absolues, des analyses de puissance, des paramètres statistiques, des fonctions de répartition et une analyse d'ondelettes des informations concernant les vibrations dans la plage de temporisation (11) sont extraits en tant que caractéristiques.

12. Procédé selon la revendication 8, où l'information concernant les vibrations dans la plage de temporisation (11) est traitée par un dispositif d'analyse des vibrations (3) et des signaux correspondants concernant les vibrations dans la plage de fréquences (12) sont définis à partir de ces dernières.

13. Procédé selon l'une quelconque des revendications 8 ou 12, où lors du traitement d'informations concernant les vibrations dans la plage de fréquences (12), une représentation des informations sous la forme d'un dénommé diagramme en cascade est utilisée, cette représentation des informations étant traitée à l'aide de procédés de traitement des images et des caractéristiques correspondantes issues des informations concernant les vibrations dans la plage de temporisation (12) sont déterminées à partir de ces dernières.

14. Procédé selon la revendication 13, où lors du traitement des informations concernant les vibrations dans la plage de fréquences (12), des observations géométriques de l'image globale ou de certaines régions de l'image sont réalisées et/ou lors du traitement des informations concernant les vibrations dans la plage de fréquences (12), des dénommées « Sky-Lines » du diagramme en cascade sont observées par ailleurs à partir de la perspective de la fréquence ou de l'axe de temps/de vitesse de rotation, et des caractéristiques correspondantes en sont extraites.

15. Procédé selon la revendication 13, où lors du traitement d'informations concernant les vibrations dans la plage de fréquences (12), les informations du diagramme en cascade sont en outre détectées de façon numérique et des méthodes issues du calcul des matrices et des vecteurs ou des procédés pour l'identification du système dans la plage de fréquences sont utilisées, ainsi qu'une analyse de répartition des données numériques, pour obtenir des informations concernant les vibrations dans la plage de fréquences (12) et/ou des fonctions de transmission.
